# EUROPÄISCHE PATENTSCHRIFT

(11) **EP 3 165 920 B1**
(45) Veröffentlichungstag und Bekanntmachung des Hinweises auf die Patenterteilung: **03.07.2019**
(21) Anmeldenummer: 15193489.0
(22) Anmeldetag: 06.11.2015
(51) Int. Cl.: G01N 33/48, G01N 33/543, G01N 33/567

(54) **VERFAHREN ZUR ANALYSE VON SERUM AUF ANTI-ERYTHROZYTÄRE ANTIKÖRPER**
METHOD FOR ANALYSIS OF SERUM ON ANTI-ERYTHROCYTE ANTIBODIES
PROCEDE D'ANALYSE DE SERUM SUR ANTI-ERYTHROCYTAIRES

(43) Veröffentlichungstag der Anmeldung: 10.05.2017
(73) Patentinhaber: imusyn GmbH & Co. KG, 30625 Hannover (DE)
(72) Erfinder: Schneeweiß, Clemens, 30173 Hannover (DE); Kellermann, Thomas, 30449 Hannover (DE); Marsollek, Anja, 30451 Hannover (DE)
(74) Vertreter: Taruttis, Stefan Georg

(56) Entgegenhaltungen:
- WO-A1-2012/010666
- WO-A2-2007/081778
- CN-A- 101 109 756
- CN-A- 101 387 648
- CN-A- 104 569 400
- OLIVIER BOUIX ET AL: "Erythrocyte-magnetized technology: an original and innovative method for blood group serology", TRANSFUSION, AMERICAN ASSOCIATION OF BLOOD BANKS, BETHESDA, MD, US, Bd. 48, Nr. 9, 1. September 2008 (2008-09-01), Seiten 1878-1885, XP002657699, ISSN: 0041-1132, DOI: 10.1111/J.1537-2995.2008.01790.X [gefunden am 2008-06-02]
- HOWON LEE ET AL: "Colour-barcoded magnetic microparticles for multiplexed bioassays", NATURE MATERIALS, Bd. 9, Nr. 9, 1. September 2010 (2010-09-01), Seiten 745-749, XP055087368, ISSN: 1476-1122, DOI: 10.1038/nmat2815
- AXEL SELTSAM ET AL: "Recombinant blood group proteins facilitate the detection of alloantibodies to high-prevalence antigens and reveal underlying antibodies: results of an international study", TRANSFUSION., Bd. 54, Nr. 7, 1. Juli 2014 (2014-07-01), Seiten 1823-1830, XP055258207, US ISSN: 0041-1132, DOI: 10.1111/trf.12553
- H G HEUFT ET AL: "Soluble recombinant blood group proteins for red blood cell antibody identification", TRANSFUS MED HEMOTHER 2014, Bd. 41, Nr. suppl 1, 1. September 2014 (2014-09-01), Seiten 18-19, XP055258220,
- K. Ridgwell ET AL: "Production of soluble recombinant proteins with Kell, Duffy and Lutheran blood group antigen activity, and their use in screening human sera for Kell, Duffy and Lutheran antibodies", TRANSFUSION MEDICINE, vol. 17, no. 5, 1 October 2007 (2007-10-01), pages 384-394, XP055446837, GB ISSN: 0958-7578, DOI: 10.1111/j.1365-3148.2007.00762.x
- Axel Seltsam ET AL: "Prokaryotic versus eukaryotic recombinant Lutheran blood group protein for antibody identification", TRANSFUSION., vol. 47, no. 9, 1 September 2007 (2007-09-01), pages 1630-1636, XP055446859, US ISSN: 0041-1132, DOI: 10.1111/j.1537-2995.2007.01334.x

## Beschreibung

Die vorliegende Erfindung betrifft ein Verfahren zur Analyse von Serum auf das Vorhandensein anti-erythrozytärer Antikörper mit dem Ziel, die Kompatibilität des Serums einer Blutprobe mit den Blutgruppenantigenen, insbesondere Antigenen von Erythrozyten, einer anderen Blutprobe zu bestimmen. Dabei kann das zu testende Serum beispielsweise von einem möglichen Empfänger des Bluts stammen, so dass mit dem Verfahren die Antikörper des Serums bestimmt werden, die mit den Blutgruppenantigenen einer Blutprobe reagieren.

### Stand der Technik

Die US 2010/0075865 A1 beschreibt die Herstellung einer Unterlage, auf der eine Vielzahl von Partikeln immobilisiert ist, die jeweils ein spezifisches Bindemolekül für einen Analyten aufweisen. Die verschiedenen Partikel werden nacheinander auf der Unterlage immobilisiert, wobei für jeden Partikel der Ort der Immobilisierung optisch aufgenommen und in Bezug zu einem Referenzpunkt auf der Unterlage gespeichert wird, um später die Zuordnung eines an einem Ort der Unterlage detektierten Signals zu dem Partikel und dessen Bindemolekül zu erlauben.

Die US2015/0203912 beschreibt, dass Partikel, die mit Analyten-spezifischen Antikörpern gekoppelt sind, zur Markierung mit Oligonukleotiden verbunden sein können, die zum Nachweis per PCR, Hybridisierung oder Sequenzierung identifiziert werden.

Die WO 2010/042745 beschreibt ein Analyseverfahren, bei dem mit einem mikroskopisch detektierbaren Code versehene Partikel mit einer spezifischen Substanz zur Analyse versehen sein können, wobei optisch verschieden codierte Partikel nebeneinander in einem Napf mit einer Probe kontaktiert werden können.

Die WO 2008/148890 beschreibt die Analyse von Antikörpern in einer Serumprobe unter Verwendung von unterscheidbaren Partikeln, auf denen Erythrozyten oder erythrozytäre Membranfragmente immobilisiert sind, mit anschließendem Nachweis daran gebundener Antikörper.

Üblich ist das Mischen einer Serumprobe in Einzelreaktionen mit jeweils einer Spezies Erythrozyten mit vorbestimmten Blutgruppenantigenen und das Zentrifugieren der Mischung durch eine Schüttung aus Gelpartikeln, das auch als Partikel-Gel-Immuntest (PaGIA) bezeichnet wird. Das Vorliegen von Antikörpern im Serum wird durch Agglutination angezeigt, die ein Durchlaufen der Erythrozyten durch die Schüttung verhindert.

Seltsam et al., Transfusion 1823-1830 (2014) beschreiben die heterologe Expression von Blutgruppenantigenen in tierischen Zellen und deren Verwendung zur Neutralisation von Antikörpern in Serum, bevor ein PaGIA mit Erythrozyten durchgeführt wird.

An der Verwendung von Erythrozyten ist nachteilig, dass die im Serum vorliegenden Antikörper indirekt aus der Kombination der auf den Erythrozyten vorliegenden Antigene herzuleiten sind und daher eine eindeutige Ableitung der Antikörper sehr von der Kombination der erythrozytären Antigene abhängt.

Bouix et al., Transfusion 1878-1885 (2008) beschreibt ein Verfahren zur Analyse von Blutserum auf Antiköper gegen die AB0-RH-K Blutgruppenantigene, bei dem in einzelnen Näpfen jeweils eine Sorte vormagnetisierter Erythrozyten mit dem zu testenden Serum gemischt wird. Aufgrund der Schichtung der vormagnetisierten Erythrozyten oberhalb einer Plasmaprobe sollen die Erythrozyten durch die Plasmaprobe wandern, dabei Antikörper annehmen, durch eine Filterschicht wandern und schließlich an dem am Boden immobilisierten anti-Mensch-IgG haften.

Die WO 2012/010666 A1 verwendet auf magnetischen Partikeln immobilisierte Erythrozyten zum Kontaktieren mit einer Serumprobe, wobei die Mischung aus Serum und Erythrozyten durch Magnetkraft durch einen trichterförmigen Bereich gezogen wird, der mit einer viskosen Lösung gefüllt ist und dessen Wände mit einem anti-Immunglobulin beschichtet sind.

Die WO 2007/081778 A2 beschreibt die Verwendung von verschiedenen Erythrozyten oder Membranvesikeln, die jeweils mit einer individuellen Fluoreszenzmarkierung versehen sind. Bei einer positiven Reaktion mit einem spezifischen Antikörper kann dieser mit einem fluoreszenzmarkierten sekundären Antikörper nachgewiesen werden und mit den farblichen Markierungen der Zellen oder Membranvesikel korreliert werden.

Nach der englischen Computerübersetzung betreffen die CN 104569400 A und CN 101387648 A die Markierung von Erythrozytenmembranen mit kolloidalem Gold zur Verwendung bei der Analyse von Serumproben jeweils in separaten Näpfen.

Nach der englischen Computerübersetzung betrifft die CN 101109756 A ein ELISA-Verfahren, bei dem gereinigte Erythrozyten in Näpfen immobilisiert sind.

Lee et al., nature materials 745-749 (2010) beschreibt die Herstellung von mit Farbstreifen markierten magnetischen Mikropartikeln für Analysen am Beispiel eines DNA - Hybridisierungstests.

Seltsam et al., Transfusion 1823-1830 (2014) und Heuft et al., Abstract in Transfus Med Hemother (2014) verwenden zur Analyse die klassischen Gelkarten mit Zusatz eines rekombinant hergestellten Blutgruppenantigens, um entsprechende Antikörper zu maskieren.

Seltsam et al., Transfusion1630-1636 (2007) und Ridgwell et al., Transfusion Medicine 384-394 (2007) beschreiben zum Nachweis von Serumantikörpern ELISA mit rekombinant hergestellten isolierten Blutgruppenantigenen.

### Aufgabe der Erfindung

Der Erfindung stellt sich die Aufgabe, ein alternatives Verfahren zur Analyse von Antikörpern in einer Serumprobe bereitzustellen, die gegen Blutguppenantigene gerichtet sind, insbesondere ein Verfahren mit hoher Genauigkeit. Bevorzugt soll das Verfahren eine höhere Empfindlichkeit aufweisen und/oder eine präzisere Bestimmung der Blutgruppenantikörper erlauben, insbesondere gegenüber dem konventionellen PaGIA.

### Beschreibung der Erfindung

Die Erfindung löst die Aufgabe mit den Merkmalen der Ansprüche, insbesondere durch ein Analyseverfahren zur Bestimmung von Antikörpern in einer Serumprobe, die gegen Blutguppenantigene (BGA) gerichtet sind,
1. bei dem ein Aliquot der Serumprobe gleichzeitig mit zumindest einem, bevorzugt mit zumindest 3 oder zumindest sechs unterschiedlichen Membranfragmenten, die zumindest ein BGA aufweisen und insbesondere Erythrozytenmembranfragmente sind, oder zumindest ein Blutgruppenantigen tragende Zellen, die insbesondere Erythrozyten sind, kontaktiert wird, deren jeweilige Blutguppenantigene vorbestimmt sind und die jeweils auf vorbestimmten, identifizierbaren Oberflächenabschnitten von Trägern immobilisiert sind, und
2. mit zumindest einem, bevorzugt zumindest drei, bevorzugter zumindest sechs unterschiedlichen isolierten BGA kontaktiert wird, die jeweils auf vorbestimmten, identifizierbaren Oberflächenabschnitten von Trägern immobilisiert sind,
3. in einem gemeinsamen Flüssigkeitsvolumen,
4. mit anschließendem Abtrennen ungebundener Antikörper von den Oberflächenabschnitten,
5. Zugeben eines mit einer detektierbaren Markierung versehenen Reagenzes, das an Antikörper des Serums bindet, beispielsweise ein optisch markierter anti-human-Antikörper,
6. Entfernen von ungebundenem Reagenz von den Oberflächenabschnitten,
7. optisches Detektieren der Markierung und Bestimmen der vorbestimmten, individuellen Oberflächenabschnitte,
8. mit anschließendem Zuordnen des Ergebnisses des Detektierens der Markierung zu den vorbestimmten individuellen Oberflächenabschnitten und
9. Auswerten des Vorliegens oder der Abwesenheit der den individuellen Oberflächenabschnitten zugeordneten detektierten Markierungen zur Bestimmung der Antikörper, insbesondere durch Vergleichen des Vorliegens oder der Abwesenheit der Markierungen, die den individuellen Oberflächenabschnitten für isolierte BGA zugeordnet sind, mit dem Vorliegen oder der Abwesenheit der Markierungen, die den individuellen Oberflächenabschnitten für Erythrozytenmembranfragmente zugeordnet sind.

Die Spezies Membranfragmente, die zumindest ein BGA aufweisen, werden vorliegend stellvertretend auch als Erythrozytenmembranfragmente bezeichnet und sind vorzugsweise Membranfragmente von Erythrozyten. Die Spezies der zumindest ein Blutgruppenantigen aufweisenden Zellen werden vorliegend stellvertretend auch als Erythrozyten bezeichnet und sind insbesondere Erythrozyten. In Schritt 1 werden zumindest drei unterschiedliche Spezies von zumindest ein Blutgruppenantigen aufweisenden Zellen und/oder Membranfragmenten, die jeweils auf vorbestimmten, identifizierbaren Oberflächenabschnitten von Trägern immobilisiert sind, gleichzeitig mit einem Aliquot der Serumprobe kontaktiert. Dabei sind die jeweiligen Blutgruppenantigene der zumindest ein Blutgruppenantigen aufweisenden Zellen und die jeweiligen Blutgruppenantigene der zumindest ein Blutgruppenantigen aufweisenden Membranfragmente vorbestimmt.

Dabei sind die Oberflächenabschnitte Partikel, die einen individuellen, lichtmikroskopisch erkennbaren Code aufweisen, so dass jedes BGA und jede Spezies von Erythrozytenmembranfragmenten auf Partikeln immobilisiert sind, die jeweils einen individuellen Code aufweisen, der mikroskopisch auslesbar bzw. bestimmbar ist.

Dabei sind die Partikel mit dem lichtmikroskopisch erkennbaren individuellen Code mit jeweils einer Spezies Erythrozytenmembranfragment bzw. einer Spezies eines BGA verbunden. Entsprechend wird jeder Spezies der Erythrozytenmembranfragmente bzw. jeder Spezies BGA ein lichtmikroskopisch erkennbarer individueller Code zugeordnet und das Ergebnis des Detektierens der Markierung. Bevorzugt sind die isolierten BGA rekombinant in einer Wirtszelle exprimierte Proteine oder Abschnitte daraus, die ein Epitop aufweisen. Alternativ können die isolierten BGA aus Zellen, die insbesondere Erythrozyten sind, gereinigte Proteine sein, oder Abschnitte solcher, die ein Epitop aufweisen. Bevorzugt sind die BGA oder deren Abschnitte lösliche Proteine. Alternativ können die isolierten BGA aus Oligo- oder/und Polysacchariden bestehen, die ein Epitop aufweisen. Bevorzugt werden diese Oligo- oder/und Polysaccharide synthetisch hergestellt oder aus Zellen, die insbesondere Erythrozyten sind, gereinigt.

Erythrozytenmembranfragmente, die auf vorbestimmten, identifizierbaren Oberflächenabschnitten von Trägern immobilisiert sind, können z.B. durch Binden von Erythrozyten an die Träger mit anschließender Lyse der Erythrozyten hergestellt werden. Zur Lyse können die Träger mit darauf gebundenen Erythrozyten z.B. mit einem hypotonischen Puffer kontaktiert werden. Zur Immobilisierung der Erythrozyten können diese z.B. mit einem Bindepartner eines Bindepaars versehen werden, z.B. biotinyliert werden, wobei der Bindepartner bevorzugt eine chemisch reaktive Gruppe aufweist, und die Träger den anderen Bindepartner des Bindepaars gebunden aufweisen, z.B. mit Streptavidin beschichtet sein.

Im Verfahren können zunächst Erythrozyten an Trägern immobilisiert werden und dann lysiert werden, um auf den Oberflächenabschnitten von Trägern immobilisierte Erythrozytenmembranfragmente zu erzeugen, mit dem anschließendem Kontaktieren mit dem Serum in Schritt 1.

Alternativ können im Verfahren in Schritt 1 Erythrozyten, die auf den Oberflächenabschnitten von Trägern immobilisiert sind, mit Serum kontaktiert werden, wobei während des Kontaktierens oder anschließend, z.B. nach Schritt 4, die immobilisierten Erythrozyten lysiert werden.

Für die Zwecke der Erfindung wird eine Spezies Erythrozytenmembranfragmente bzw. eine Spezies BGA auch als isoliertes Erythrozytenmembranfragment bzw. als isoliertes BGA bezeichnet.

Generell bevorzugt sind die Oberflächenabschnitte der Träger, auf denen die BGA bzw. Erythrozytenmembranfragmente immobilisiert sind, in ihrer Beschaffenheit gleich, so dass sie nur dadurch vorbestimmt identifizierbar sind, dass sie mit einem individuellen Code versehen sind.

Die Oberflächenabschnitte sind durch einen mikroskopisch auslesbaren optischen Code, der aus einem Muster aus hellen und dunklen Flächen besteht, identifizierbar.

Zusätzlich können die Oberflächenabschnitte durch eine vorbestimmte und daher bekannte Lage bzw. Stelle individuell vorbestimmt sein, so dass das Detektieren der Markierung an den vorbestimmten Stellen des Trägers erfolgt und sich bereits daraus eine Zuordnung der Detektion zu den bestimmten Oberflächenabschnitten ergibt. In dieser Ausführungsform können die Oberflächenabschnitte auf einem gemeinsamen Träger liegen, bzw. individuelle Oberflächenabschnitte eines gemeinsamen Trägers sein. Ein solcher Träger kann z.B. plattenförmig sein, wobei die BGA bzw. Erythrozytenmembranfragmente an jeweils vorbestimmten Stellen immobilisiert sind.

Das Reagenz, das an Antikörper des Serums bindet, ist bevorzugt ein anti-Human-Antikörper. Die detektierbare Markierung ist bevorzugt eine optisch detektierbare Markierung und kann eine unmittelbar optisch detektierbare Markierung sein, z.B. ein fluoreszierendes oder anderweitig optisch aktives Molekül, oder eine mittelbar optisch detektierbare Markierung, z.B. ein Enzym, das ein zusätzlich zugegebenes Substrat zu einer farbigen Verbindung umsetzt. Alternativ kann die detektierbare Markierung ein Oligonukleotid sein und das Detektionsverfahren zur Identifikation des Oligonukleotids eine PCR, Hybridisierung oder Sequenzierung sein. Für die Zwecke der Erfindung werden auch Oligonukleotide als optisch detektierbar bezeichnet, einschließlich einer Detektion mittels PCR oder Hybridisierung oder einer Detektion mittels ionenempfindlicher Feldeffekttransistoren bei der Sequenzbestimmung von Oligonukleotiden.

Bevorzugt wird für den Fall, dass bei allen immobilisierten Erythrozytenmembranfragmenten, bzw. für deren vorbestimmte individuelle Oberflächenabschnitte, insbesondere für die lichtmikroskopisch bestimmbaren Codes von partikelförmigen Oberflächenabschnitten, die mit Erythrozytenmembranfragmenten verbunden sind, das Vorliegen der Markierung des Reagenzes detektiert wird, zu einem weiteren Aliquot der Serumprobe zumindest ein BGA als lösliches BGA zugegeben, das z.B. ausgewählt ist aus der Gruppe, die Kn(a)/McC(a), Yt(a), Cr(a)/Dr(a), C4B^{∗}3, C4A^{∗}3, Sc1, JMH, Ch(a), Fy(a), Fy(b), Kell, Cellano, Do(a), Do(b), Lu(a), Lu(b), Xg(a), In(b), LW(a) und/oder eines der Antigene umfasst oder daraus besteht, die an einem Oberflächenabschnitt gebunden sind, für den das Vorliegen der optisch detektierbaren Markierung gemessen wurde, und anschließend die Schritte 1 bis 8 erneut durchgeführt werden. Bevorzugt wird ein Aliquot des Serums, optional generell vor Schritt 1, mit zumindest einem der vorgenannten BGA inkubiert, vorzugsweise mit einer Kombination aus Kn(a)/McC(a), Yt(a) und Cr(a)/Dr(a) oder einer Kombination aus Yt(a), C4B^{∗}3, C4A^{∗}3, Sc1 und JMH. Denn Antikörper gegen diese BGA sind klinisch nicht relevant und können daher mit dem zugesetzten BGA blockiert werden, ohne klinisch bedeutende Antikörper auszuschalten. Bevorzugt ist das zugesetzte BGA ebenso wie das zumindest eine immobilisierte BGA ein lösliches Protein, z.B. ein ohne eine Membranbindedomäne rekombinant hergestelltes BGA, oder ein lösliches Oligo- und/oder Polysaccharid, das ein BGA bildet.

In einer bevorzugten Ausführungsform liegen die Oberflächenabschnitte in Mischung mit zumindest einem, vorzugsweise allen der vorgenannten BGA vor, gegen die Antikörper klinisch nicht relevant sind. Z.B. kann das zumindest eine, vorzugsweise alle dieser BGA in wässriger Lösung in Mischung mit den Oberflächenabschnitten vorliegen. Bevorzugt liegen die Oberflächenabschnitte in getrockneter Form vor, wobei bevorzugt der zumindest eine, vorzugsweise alle dieser BGA an den getrockneten Oberflächenabschnitten anhaften, z.B. in getrockneter Form, z.B. gefriergetrocknet. Oberflächenabschnitte in getrockneter Form werden vor dem Verfahren in einer wässrigen Zusammensetzung und/oder bei dem Verfahren mit dem Serum kontaktiert, so dass die daran anhaftenden BGA für klinisch nicht relevante Antikörper in Lösung gehen können. In dieser Ausführungsform können die Oberflächenabschnitte dadurch hergestellt sein, dass sie nach dem Immobilisieren von isolierten Erythrozytenmembranfragmenten oder isolierten BGA in wässriger Lösung mit zumindest einem, vorzugsweise allen dieser BGA für klinisch nicht relevante Antikörper gemischt werden und getrocknet werden, z.B. gefriergetrocknet. Dabei können die Oberflächenabschnitte, auf denen jeweils isolierte Erythrozytenmembranfragmente oder isolierte BGA immobilisiert sind, jeweils einzeln oder in einer Mischung mit zumindest einem weiteren Oberflächenabschnitt mit einem anderen isolierten Erythrozytenmembranfragment oder isolierten BGA in wässriger Mischung mit zumindest einem, vorzugsweise allen dieser BGA getrocknet werden, gegen die Antikörper klinisch nicht relevant sind. Im Verfahren können solche getrockneten Oberflächenabschnitte in wässrigem Medium aufgenommen werden, so dass darauf getrocknete BGA für klinisch nicht relevante Antikörper in dem wässrigen Medium verteilt werden, oder solche getrockneten Oberflächenabschnitte können direkt mit einem Aliquot der Serumprobe kontaktiert werden.

Die Zugabe eines löslichen Antigens, insbesondere eines der Antigene, für die das Vorliegen der Markierung durch Zuordnung zu dem vorbestimmten und individuellen Oberflächenabschnitt des gebundenen Partikels detektiert wurde, erlaubt eine präzisere Bestimmung der in dem Serum vorliegenden Antikörper. Denn das Vorinkubieren eines Aliquots des Serums mit einem löslichen Antigen führt zur anschließenden Blockierung der dafür spezifischen Antikörper bei der anschließenden Kontaktierung dieses weiteren Aliquots des Serums mit der Kombination von Oberflächenabschnitten, die immobilisierte Erythrozytenmembranfragmente oder ein isoliertes BGA aufweisen. Für den Fall, dass die Zugabe von einem löslichen Blutgruppenantigen zu dem Aliquot der Serumprobe nicht zu einer Verminderung der Anzahl der Markierungen, insbesondere bei den Oberflächenabschnitten, die mit Erythrozytenmembranfragmenten verbunden sind, führt, wird die Zugabe eines löslichen Antigens bevorzugt mit einem anderen löslichen BGA durchgeführt.

Zur Bestimmung der gegen Blutgruppenantigene gerichteten Antikörper der Serumprobe wird zuerst für die Oberflächenabschnitte bzw. deren Codes, denen eine Spezies eines isolierten BGA zugeordnet ist, das Vorliegen eines Antikörpers festgelegt, denen eine detektierte Markierung zugeordnet wurde und die Abwesenheit eines Antikörpers für die Oberflächenabschnitte, denen keine detektierte Markierung zugeordnet wurde. Anschließend werden die vorliegenden und abwesenden Antikörper mit den Oberflächenabschnitten, denen Erythrozytenmembranfragmente zugeordnet sind, verglichen. Generell werden gegen Blutgruppenantigene gerichtete Antikörper für die Zwecke der Erfindung auch nur als Antikörper bezeichnet, da sich die Erfindung auf den Nachweis solcher Antikörper bezieht. Für den Fall, dass nur für diejenigen Oberflächenabschnitte bzw. Codes oder vorbestimmten, individuellen Stellen mit Erythrozytenmembranfragmenten eine Markierung detektiert wurde, die ein BGA aufweisen, für das bei den isolierten BGAs eine Markierung detektiert wurde, werden durch die Erythrozytenmembranfragmente nur die Antikörper für BGAs identifiziert, für die mit den isolierten BGAs das Vorliegen eines Antikörpers festgelegt wurde. Um einen Antikörper zu identifizieren, der gegen ein BGA gerichtet ist, das nur auf den Erythrozytenmembranfragmenten, jedoch nicht als isoliertes BGA im Verfahren analysiert wird, werden die auf den Erythrozytenmembranfragmenten vorliegenden Kombinationen von BGAs verglichen, wobei diejenigen ausgeschlossen werden, für die auf anderen Erythrozytenmembranfragmenten oder als isolierte BGAs keine Markierung detektiert wird.

Für den Fall, dass nicht für alle Oberflächenabschnitte mit Erythrozytenmembranfragmenten eine Markierung detektiert wurde, für deren BGAs bei den isolierten BGAs eine Markierung detektiert wurde, wird bei der Auswertung bevorzugt davon ausgegangen, dass ein Antikörper gegen dieses BGA vorliegt. Denn im Verfahren ist die Empfindlichkeit für Antikörper für die isolierten BGA, die auf Oberflächenabschnitten immobilisiert sind, in den bisher beobachteten Fällen höher als die Empfindlichkeit für die immobilisierten Erythrozytenmembranfragmente, die dieses BGA aufweisen. Weiter bevorzugt wird in einem solchen Fall das Verfahren mit einem Aliquot wiederholt, wobei das BGA, für das eine Markierung detektiert wurde, in löslicher Form zugegeben wird. Dabei zeigt das Unterdrücken der Detektion einer Markierung für den Oberflächenabschnitt mit demselben isolierten BGA das Vorliegen des dafür spezifischen Antikörpers in dem Serum.

Für den Fall, dass für die isolierten BGAs keine Markierung detektiert wird, sondern nur für zumindest ein Erythrozytenmembranfragment eine Markierung detektiert wird, kann zunächst geschlossen werden, dass in dem Serum keine Antikörper gegen die isolierten BGAs enthalten sind. In diesem Fall werden Antikörper gegen BGAs im Serum identifiziert, die nur auf den Erythrozytenmembranfragmenten vorliegen, für die eine Markierung detektiert wurde.

Bevorzugt wird bei der Bestimmung der Detektion einer Markierung an einem Oberflächenabschnitt von dem Messsignal ein Messwert als unspezifischer Hintergrund subtrahiert oder anders verrechnet. Der als unspezifischer Hintergrund gemessene Messwert ist z.B. derjenige, der um den Faktor von zumindest 3 unter dem höchsten Messwert liegt und der bevorzugt zusätzlich der niedrigste Messwert ist. Der als unspezifischer Hintergrund gemessene Messwert kann derjenige sein, der für einen Oberflächenabschnitt mit isoliertem BGA gemessen wird, z.B. einen Oberflächenabschnitt, auf dem isoliertes, nicht-menschliches BGA immobilisiert ist oder nicht-menschliche Erythrozytenmembranfragmente immobilisiert sind, die nicht mit menschlichen Antikörpern reagieren. Der als unspezifischer Hintergrund gemessene Messwert kann derjenige sein, der für Oberflächenabschnitte gemessen wird, die mit einem Reagenz beschichtet sind, für das keine menschlichen Antikörper im Serum vorliegen können, z.B. mit einem menschlichen Protein, insbesondere mit humanem Serumalbumin beschichtete oder unbeschichtete Partikel. Der Hintergrund kann alternativ oder zusätzlich z.B. der Messwert für ein antithetisches Antigen sein, wenn für das betrachtete Antigen ein höherer Messwert, bevorzugt ein signifikant höherer Messwert gemessen wird, z.B. der für Fy(a) gemessen wird, wenn für Fy(b) ein höherer Messwert, bevorzugt ein signifikant höherer Messwert gemessen wird, bzw. der Messwert, der für Fy(b) gemessen wird, wenn für Fy(a) ein höherer Messwert, bevorzugt ein signifikant höherer Messwert gemessen wird. Das antithetische BGA kann z.B. ausgewählt sein aus dem Paar Fy(a) und Fy(b), dem Paar Kell und Cellano, dem Paar Lutheran (a) und Lutheran (b) und dem Paar Dombrock (a) und Dombrock (b).

Die Partikel, die einen lichtmikroskopisch bestimmbaren individuellen Code aufweisen, sind solche, die als Code ein individuelles Muster aus hellen und dunklen Flächen aufweisen, beispielsweise solche, die in der WO 2010/042745 beschrieben sind.

Die Blutgruppenantigene sind vorzugsweise rekombinant hergestellte und gereinigte Blutgruppenantigene oder Abschnitte dieser. Abschnitte von Blutgruppenantigenen weisen bevorzugt das Epitop oder die Epitope des natürlichen BGA auf.

Die Erythrozytenmembranfragmente weisen eine vorbestimmte Kombination von Blutgruppenantigenen auf, wobei die Kombination von Blutgruppenantigenen beispielsweise durch genetische Analyse des Spenders vorbestimmt sein kann.

Die Auswahl an Erythrozytenmembranfragmenten sollte vorzugsweise folgende BGA einschließen: Rh Antigene (C, Cw, c, D, E, e), K (Kell), k (Cellano), Fy(a) (Duffy (a)), Fy(b) (Duffy (b)), Jk(a) (Kidd (a)), Jk(b) (Kidd (b)), MNS Antigene (S, s, M, N), P(1), Le(a) (Lewis(a)), Le(b) (Lewis(b)), wobei von dieser Auswahl vorzugsweise folgende Merkmale homozygot vorliegen sollten: Rh Antigene D und c, Fy(a), Fy(b), Jk(a), Jk(b), MNS Antigene S und s. Das erfindungsgemäße Verfahren hat den Vorteil, dass die Kombination von individuellen BGAs und Erythrozytenmembranfragmenten, die naturgemäß mehrere BGAs aufweisen, die in der Serumprobe vorliegenden Antikörper gegen Blutgruppenantigene mit höherer Genauigkeit feststellen lässt, als beispielsweise die Analyse nur mit Erythrozytenmembranfragmenten. Denn die Analyse der Bindung von Antikörpern aus dem Serum an individuelle BGA erlaubt eine direkte und eindeutige Bestimmung des Vorliegens eines Antikörpers gegen dieses BGA.

Dadurch, dass das Analyseverfahren in einem Volumen abläuft, in dem gleichzeitig ein Aliquot der Serumprobe und die an vorbestimmte, individuelle Oberflächenabschnitte gebundenen Erythrozytenmembranfragmente und isolierten BGA vorliegen, hat den Vorteil, dass Pipettierfehler sicherer erkannt werden, als bei jeweils getrennten Reaktionen einzelner Erythrozytenmembranfragmente oder BGA. So wird die fehlende Zugabe von Serum oder des an humane Antikörper bindenden Reagenzes oder einer Komponente einer Nachweisreaktion für die Markierung des Reagenzes zur Abwesenheit jeglichen positiven Signals führen und damit auf eine fehlerhafte Durchführung des Verfahrens anzeigen.

Überraschenderweise hat sich herausgestellt, dass das Verfahren eine niedrigere Nachweisgrenze bzw. eine bessere Auflösung besitzt als die herkömmliche Analyse durch den Partikel-Gel-Immuntest (PaGIA).

Die Erfindung wird nun genauer an Hand von Beispielen und mit Bezug auf die Figuren beschrieben, die in
- Fig. 1 schematisch den bevorzugten Ablauf der Bestimmung der Antikörper aus der Detektion der Markierungen,
- Fig. 2 eine Gelkarte eines PaGIA für eine Verdünnungsreihe eines Serums mit Erythrozytenmembranfragmenten, die Cr(a)/ Dr(a) als BGA aufwiesen,
- Fig. 3 die Messwerte der Verdünnungsreihe des Serums von Fig. 2 im erfindungsgemäßen Verfahren mit Cr(a)/ Dr(a) als isoliertes BGA,
- Fig. 4 eine Gelkarte eines PaGIA für eine Verdünnungsreihe eines Serums mit Erythrozytenmembranfragmenten, die Lu(a) als BGA aufwiesen, und in
- Fig. 5 die Messwerte der Verdünnungsreihe des Serums von Fig. 4 im erfindungsgemäßen Verfahren mit Lu(a) als isoliertes BGA zeigen.

Dabei werden stellvertretend für vorbestimmte, individuelle Oberflächenabschnitte von Trägern Partikel verwendet, die zur vorbestimmten Individualisierung für jedes BGA bzw. jedes Erythrozytenmembranfragment mit einem anderen lichtmikroskopisch auslesbaren Code versehen sind und die paramagnetisch sind. Der Code besteht aus einem Muster heller und dunkler Flächenabschnitte.

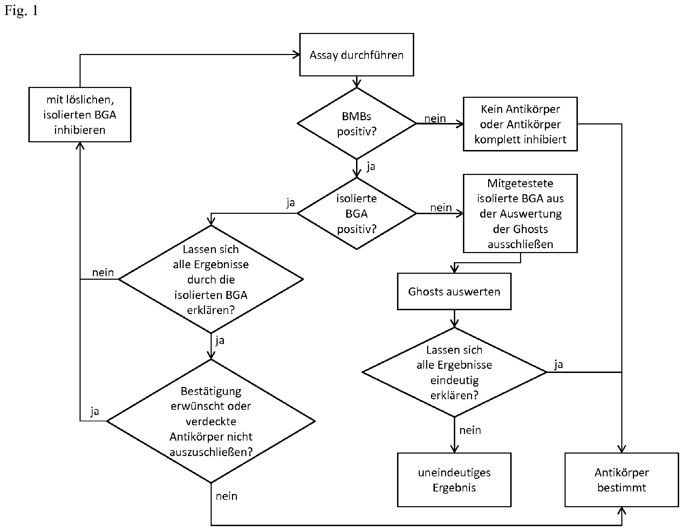

Fig. 1 zeigt eine Übersicht der bevorzugten Schritte des Auswertens des Ergebnisses des Detektierens. Zunächst sind nach den Schritten 1 bis 8 ("Assay durchführen") das Vorliegen oder die Abwesenheit der Markierung ("BMBs positiv?") den Codes zugeordnet, mit denen die Partikel (BMB) versehen sind, die jeweils eine Spezies eines immobilisierten BGA oder eines immobilisierten Erythrozytenmembranfragments aufweisen. Das Vorliegen der Detektion der Markierung, mit dem das gegen Antikörper gerichtete Reagenz versehen ist, zeigt daher das Vorliegen eines Antikörpers an, die Abwesenheit der Markierung die Abwesenheit eines Antikörpers. In der Folge ist das Vorliegen oder die Abwesenheit eines Antikörpers aus dem Serum den auf den Partikeln jeweils immobilisierten BGA oder Erythrozytenmembranfragmenten zugeordnet. Die BGA sind bevorzugt rekombinant hergestellte Blutgruppenantigene (rBGA). Die Abwesenheit jeglicher Markierung ("BMBs positiv?" - nein) zeigt, dass für die Antigene der BGA und der Erythrozytenmembranfragmente im Serum keine Antikörper vorliegen oder die im Serum enthaltenen Antikörper inhibiert sind ("Kein Antikörper oder Antikörper komplett inhibiert"), z.B. durch Zugabe zumindest eines löslichen Antigens.

Als isolierte BGA wurde jeweils eine Spezies eines BGA eingesetzt, die rekombinant hergestellt und gereinigt wurde, oder die aus Erythrozyten gereinigt wurde. Isolierte BGA, die aus Oligo- oder/und Polysacchariden bestehen, können synthetisiert werden. Für den Fall, dass zumindest eine Markierung für einen Partikel detektiert wird ("BMBs positiv?" - ja), wird bevorzugt geprüft ob dies das Vorliegen einer Markierung an einem isolierten BGA ("isolierte BGA positiv?") ist. Denn dies zeigt eindeutig das Vorliegen eines Antikörpers gegen dieses BGA an. Für den bejahenden Fall ("isolierte BGA positiv?" - ja) wird geprüft, ob das Vorliegen von Antikörpern an den isolierten BGA mit den für die Erythrozytenmembranfragmente detektierten Markierungen übereinstimmt ("Lassen sich alle Ergebnisse durch die isolierten BGA erklären?"). Das Vorliegen und die Abwesenheit der Antikörper an den isolierten BGA stimmt mit den für die Erythrozytenmembranfragmente detektierten Markierungen überein, wenn auch für die Erythrozytenmembranfragmente, die diese BGA aufweisen, das Vorliegen bzw. die Abwesenheit von Antikörpern detektiert wird. Im bejahenden Fall ("Lassen sich alle Ergebnisse durch die isolierten BGA erklären?" - ja) kann eine Bestätigung oder eine Prüfung auf verdeckte Antikörper erfolgen ("Bestätigung erwünscht oder verdeckte Antikörper nicht auszuschließen?"), indem zu einem weiteren Aliquot des Serums zumindest ein BGA zugesetzt wird ("mit löslichen, isolierten BGA inhibieren"), das insbesondere eines ist, für das eine Markierung detektiert wurde, z.B. an einem isolierten BGA. Anschließend wird das Verfahren erneut durchgeführt und bei der Auswertung des Ergebnisses wird angenommen, dass ein für das zugesetzte BGA spezifischer Antikörper vorliegt. Ein verdeckter Antikörper ist z.B. einer, der für ein BGA spezifisch ist, das auf den Erythrozytenmembranfragmenten vorkommt, nicht jedoch als isoliertes BGA auf einem Partikel. Ein verdeckter Antikörper kann dadurch identifiziert werden, dass auch bei Hemmung anderer Antikörper durch zumindest ein zugesetztes lösliches BGA das Vorliegen eines Antikörpers an den Erythrozytenmembranfragmenten detektiert wird, das ein BGA trägt, das nicht als isoliertes BGA vorhanden ist.

Für den Fall, dass für die isolierten BGAs, die einzeln auf einem Oberflächenabschnitt immobilisiert sind, kein Antikörper detektiert wird ("isolierte BGA positiv?" - nein), kann das Vorliegen von Antikörpern gegen diese BGAs ausgeschlossen werden und es werden nur die davon unterschiedlichen BGA der Erythrozytenmembranfragmente (Ghost) berücksichtigt ("Mitgetestete isolierte BGA aus der Auswertung der Ghosts ausschließen"). Dann kann durch Vergleich der vorliegenden und der abwesenden Kombinationen von BGA, die auf den Erythrozytenmembranfragmenten vorliegen, auf die Antikörper rückgeschlossen werden.

Wenn sich das Vorliegen und die Abwesenheit von Markierungen für die Erythrozytenmembranfragmente und abzüglich der gegen die isolierten BGAs gerichteten Antikörper eindeutig in Übereinstimmung bringen lassen ("Lassen sich alle Ergebnisse eindeutig klären?" - ja), dann sind alle Antikörper bestimmt. Für den Fall, dass der Vergleich der vorliegenden und der abwesenden Kombinationen von BGAs, die auf den Erythrozytenmembranfragmenten vorliegen, nicht eindeutig durch bestimmte Antikörper erklärt werden kann ("Lassen sich alle Ergebnisse eindeutig klären?" - nein), werden gegebenenfalls nicht alle Antikörper identifiziert ("uneindeutiges Ergebnis").

### Beispiel 1: Nachweis von Antikörpern in Serum

Auf paramagnetischen Mikropartikel, die mit einem mikroskopisch sichtbaren Code versehen waren (Barcoded Magnetic Beads (BMB), erhältlich von der Firma Applied BioCode, Santa Fe Springs, USA) wurde jeweils ein rekombinant hergestelltes lösliches Antigen (erhältlich von Imusyn GmbH & Co. KG, Hannover, Deutschland) oder eine Spezies von Erythrozytenmembranfragmenten oder ein Kontrollprotein (Rinderserumalbumin, RSA) immobilisiert. Das jeweilige Antigen wurde nach den Anweisungen des Herstellers der Partikel auf diesen immobilisiert.

Die Erythrozytenmembranfragmente, die vorbestimmte Antigene aufwiesen (erhältlich von BIO-RAD) wurden zu einer 10%igen Suspension verdünnt und mit 50 µM EZ Link Sulfo-NHS-LC-Biotin (erhältlich von Thermo Fisher Scientific, Waltham, MA USA) für 30 min bei Raumtemperatur inkubiert. Die so biotinylierten Erythrozyten wurden anschließend dreimal mit je 600 µl 100 mM Glycin in PBS gewaschen. Die gewaschenen Erythrozyten wurden auf 5 % in PBS eingestellt und durch Inkubieren mit Streptavidin beschichteten BMB (ebenfalls erhältlich von der Firma Applied BioCode, Santa Fe Springs, USA), für 45 min bei Raumtemperatur unter Schütteln immobilisiert (100 BMB pro µl Erythrozytensuspension). Nicht gebundene Erythrozyten wurden durch Waschen mit PBS entfernt. Die BMB wurden abschließend in BWL-Puffer (1 % BSA, 0,05 % ProClin300 in PBS, pH 7,4) aufgenommen..

Für das Analyseverfahren wurde ein Serum, das bekanntermaßen anti-RhD-Antikörper und anti-Keil-Antikörper (Titer 1:32) enthält, 1:2 in 0,9 % NaCl-Lösung verdünnt und mit den oben beschriebenen BMB, die jeweils ein isoliertes BGA oder Erythrozytenmembranfragmente aufwiesen, in einem Napf einer Mikrotiterplatte für 60 min bei 37°C unter Schütteln kontaktiert. Anschließend wurden die BMB in den Näpfen dreimal mit je 200 µl PBS gewaschen. Die nachfolgende Tabelle nennt die isolierten BGA und die Antigene der jeweiligen Erythrozytenmembranfragmente (Ghost), sowie die mit dem Verfahren für die jeweiligen Codes detektierten Fluoreszenzwerte.

Als Reagenz, das an Antikörper des Serums bindet, wurde ein anti-human-Antikörper IgG-PE, 1:2000 in BWL verdünnt (erhältlich von Jackson ImmunoResearch Europe Ltd., Suffolk, Großbritannien), verwendet, der als Markierung mit dem Fluoreszenzfarbstoff Phycoerythrin (PE) gekoppelt ist. 100 µl dieser Lösung wurden für 60 min bei 37°C unter Schütteln mit den BMB kontaktiert. Daraufhin wurden die BMB im Napf dreimal mit je 200 µl PBS gewaschen. Abschließend wurden die BMB in einen hypotonischen Puffer (0,067 x PBS) aufgenommen, der die auf den BMB immobilisierten Erythrozyten fragmentiert und Erythrozytenmembranfragmente erzeugt, und die BMB in den Näpfen der Mikrotiterplatte wurden lichtmikroskopisch und fluoreszenzmikroskopisch mittels eines Biocode2000 Analyzers (erhältlich von der Firma Applied BioCode, Santa Fe Springs, USA) mit Anregung bei 530 nm und Detektion bei 575 nm detektiert. Die Zuordnung der mikroskopisch bestimmten Codes zu den detektierten Fluoreszenzwerten erfolgte computergesteuert anhand der Position der Partikel in der Mikrotiterplatte.

Die nachfolgende Tabelle nennt die isolierten BGA und die Antigene der jeweiligen Erythrozytenmembranfragmente, sowie die mit dem Verfahren für die jeweiligen Codes detektierten Fluoreszenzwerte (mittlere Fluoreszenzintensität, MFI). Als positiv wurden alle Reaktionen bewertet, deren MFI mindestens doppelt so hoch wie die MFI der Kontrolle (RSA) war. Alle Reaktionen unterhalb dieser Schwelle wurden als negativ bewertet.

| BMB Code Nr. | Antigen | MFI | Bewertung |
|---|---|---|---|
| 32 | Fy(a), isoliertes BGA | 175 | Negativ |
| 33 | Fy(b), isoliertes BGA | 153 | Negativ |
| 50 | Erythrozytenmembranfragment, RhD, Fy(a) und Fy(b) tragend, Kell negativ. Weitere Antigene sind vorhanden, für das Beispiel aber nicht relevant. | 3102 | Positiv |
| 51 | Erythrozytenmembranfragment, RhD und Fy(a) tragend, Kell und Fy(b) negativ. Weitere Antigene sind vorhanden, für das Beispiel aber nicht relevant. | 4134 | Positiv |
| 52 | Erythrozytenmembranfragment, Kell und Fy(b) tragend, RhD und Fy(a) negativ. Weitere Antigene sind vorhanden, für das Beispiel aber nicht relevant. | 938 | Positiv |
| 55 | Erythrozytenmembranfragment, RhD und Kell negativ, Fy(a) und Fy(b) tragend. Weitere Antigene sind vorhanden, für das Beispiel aber nicht relevant. | 218 | Negativ |
| 58 | Cellano, isoliertes BGA | 152 | Negativ |
| 59 | Kell, isoliertes BGA | 11333 | Positiv |
| 63 | RSA (Negativkontrolle) | 194 | Referenz |

Die detektierten Markierungen auf den BMB zeigen das Vorliegen von Antikörpern im Serum gegen die Antigene auf den BMB mit den Codes 50, 51, 52,und 59, sowie die Abwesenheit von Antikörpern gegen die Antigene auf den BMB mit den Codes 32, 33, 55 und 58.

Anhand dieses Reaktionsmusters wurde eine Auswertung gemäß dem Flussschema von Fig. 1 durchgeführt. Anti-Fy(a) oder anti-Fy(b) Antikörper können aufgrund der negativen Reaktion der BMB mit den Codes 32 und 33 ausgeschlossen werden. Das einzige positiv bewertete BMB mit isolierten BGA trägt Kell. Das einzige Erythrozytenmembranfragmente tragende BMB mit Kell (Code 52) ist ebenfalls positiv. Die Reaktionen auf den Erythrozytenmembranfragmente tragenden BMB mit den Codes 50 und 51 lassen sich jedoch nicht mit Kell erklären.

Der Nachweis des anti-RhD Antikörpers erfolgt daher gemäß dem Flussschema durch spezifische Inhibition des anti-Keil Antikörpers mit löslichem Kell. Dafür wird der identische Assay nochmals durchgeführt, das Serum jedoch vor Kontaktierung mit den BMB für 30 min bei Raumtemperatur mit 100 µg/ml löslichem Kell inkubiert.

Das so durchgeführte Verfahren ergibt folgendes Reaktionsmuster:

| BMB Code Nr. | Antigen | MFI | Bewertung |
|---|---|---|---|
| 32 | Fy(a), isoliertes BGA | 66 | Negativ |
| 33 | Fy(b), isoliertes BGA | 83 | Negativ |
| 50 | Erythrozytenmembranfragment, RhD, Fy(a) und Fy(b) tragend, Kell negativ. Weitere Antigene sind vorhanden, für das Beispiel aber nicht relevant. | 2749 | Positiv |
| 51 | Erythrozytenmembranfragment, RhD und Fy(a) tragend, Kell und Fy(b) negativ. Weitere Antigene sind vorhanden, für das Beispiel aber nicht relevant. | 3531 | Positiv |
| 52 | Erythrozytenmembranfragment, Kell und Fy(b) tragend, RhD und Fy(a) negativ. Weitere Antigene sind vorhanden, für das Beispiel aber nicht relevant. | 146 | Negativ |
| 55 | Erythrozytenmembranfragment, RhD und Kell negativ, Fy(a) und Fy(b) tragend. Weitere Antigene sind vorhanden, für das Beispiel aber nicht relevant. | 120 | Negativ |
| 58 | Cellano, isoliertes BGA | 90 | Negativ |
| 59 | Kell, isoliertes BGA | 164 | Negativ |
| 63 | RSA (Kontrolle) | 100 | Referenz |

Die detektierten Markierungen auf den BMB zeigen das Vorliegen von reaktiven Antikörpern im Serum gegen die Antigene auf den BMB mit den Codes 50 und 51, sowie die Abwesenheit von Antikörpern gegen die Antigene auf den BMB mit den Codes 32, 33, 52, 55, 58 und 59.

Das Flussschema aus Fig. 1 wird für dieses Ergebnis erneut durchlaufen. Diesmal sind keine BMB mit isolierten BGA positiv, so dass nur die Erythrozytenmembranfragmente tragenden BMB zur Auswertung herangezogen werden. Die erhaltenen Ergebnisse lassen sich eindeutig mit einem anti-RhD Antikörper erklären. Die beiden Antikörper des Serums, anti-Kell und anti-RhD, sind somit eindeutig bestimmt.

### Beispiel 2: Nachweis von Antikörpern gegen Cr(a)/Dr(a) und Lu(a)

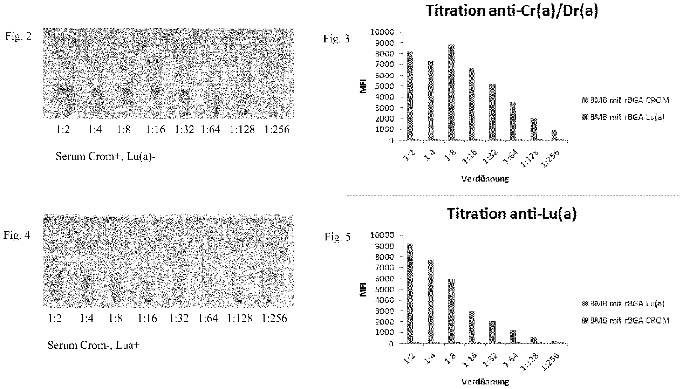

Als Beispiel für ein Serum wurde ein menschliches Serum eingesetzt, das bekanntermaßen Antikörper gegen das Antigen Cr(a)/Dr(a) und eines, das Antikörper gegen das Antigen Lu(a) enthält. Die Seren wurden jeweils in einer Verdünnungsreihe von 1:2, 1:4, 1:8, 1:16, 1:32, 1:64, 1:128 und 1:256 in Puffer eingesetzt. Zum Vergleich wurden Aliquots der Verdünnungsreihe mit Erythrozyten gemischt, die das Antigen Cr(a)/Dr(a) bzw. Lu(a) aufweisen: Die Reaktion wurde im Partikel-Gel-Immuntest als Agglutination gemessen. Die Gelkarte des Immuntests mit dem Serum, das Antikörper gegen Cr(a)/Dr(a) enthält, ist in Figur 2, die Gelkarte des Immuntests mit dem Serum, das Antikörper gegen Lu(a) zeigt, in Figur 4. Die Ergebnisse machen deutlich, dass die Agglutination durch das Serum mit anti-Cr(a)/Dr(a)-Antikörpern noch schwach bis zu einer Verdünnung von 1:128 erkennbar ist, für das Serum mit anti-Lu(a) noch schwach bis zu einer Verdünnung von 1:16.

Auf jeweils unterschiedlich optisch kodierten Partikeln wurde rekombinant exprimiertes und gereinigtes lösliches Antigen Cr(a)/Dr(a) (auch als CROM bezeichnet) oder Lu(a) (erhältlich von imusyn GmbH & Co. KG, Hannover, Deutschland) wie in Beispiel 1 immobilisiert. Die Partikel waren paramagnetische Mikropartikel, die mit einem mikroskopisch sichtbaren Code versehen waren (BMB, Applied Biocode). Das jeweilige Antigen wurde kovalent auf diesen immobilisiert. Als Hintergrundkontrolle diente jeweils das andere Antigen, gegen die in den Serumproben keine Antikörper vorlagen.

Die Spezies der Partikel wurden getrennt mit Aliquots der Verdünnungsreihe kontaktiert, gewaschen und mit fluoreszenz-markiertem anti-human-Antikörper (anti-human IgG-PE) kontaktiert. Nach Waschen wurden die Partikel in einer Mikrotiterplatte mit Flachboden lichtmikroskopisch und fluoreszenzmikroskopisch mittels eines Biocode2000 Analyzers (erhältlich von der Firma Applied BioCode) mit Anregung bei 530nm und Detektion bei 575nm (spezifisch für Phycoerythrin (PE)) detektiert. Die Zuordnung der mikroskopisch bestimmten Codes zu den detektierten Fluoreszenzwerten erfolgte computergesteuert anhand der Position der Partikel in der Mikrotiterplatte. Die Fluoreszenzintensitäten, die für die mit Cr(a)/Dr(a) oder Lu(a) beschichteten Partikel detektiert wurden, sind in Figur 3 bzw. 5 gezeigt.

Die Werte machen deutlich, dass für die Partikel der jeweiligen Hintergrundkontrolle (Lu(a) in Figur 3, CROM (Cr(a)/Dr(a)) in Figur 5) in jeder Verdünnung etwa das gleiche nur sehr schwache Signal detektiert wurde. Für die Partikel mit Cr(a)/Dr(a) wurde ein deutlich oberhalb des Hintergrunds liegendes Signal auch noch bei der höchsten gemessenen Verdünnung von 1:256 gemessen, für die Partikel mit Lu(a) noch bei einer Verdünnung von 1:256.

Im Vergleich zum Partikel-Gel-Immuntest zeigt das erfindungsgemäße Verfahren für die Partikel mit isolierten BGA eine deutlich höhere Empfindlichkeit für den Nachweis von Antikörpern, vorliegend für Cr(a)/Dr(a) eine mindestens um den Faktor 2 höhere Empfindlichkeit, für Lu(a) eine mindestens um den Faktor 16 höhere Empfindlichkeit.

## Patentansprüche

1. Analyseverfahren zur Bestimmung von gegen Blutguppenantigene gerichteten Antikörpern in einer Serumprobe mit den Schritten
1. Kontaktieren eines Aliquots der Serumprobe gleichzeitig mit zumindest einer Spezies von zumindest ein Blutgruppenantigen aufweisenden Zellen und/oder Membranfragmenten, die zumindest ein Blutgruppenantigen aufweisen, deren jeweilige Blutgruppenantigene vorbestimmt sind und die jeweils auf vorbestimmten, individuellen Oberflächenabschnitten von Trägern immobilisiert sind und
2. mit zumindest einem isolierten Blutgruppenantigen, das auf zumindest einem vorbestimmten, individuellen Oberflächenabschnitt von Trägern immobilisiert ist,
3. in einem gemeinsamen Flüssigkeitsvolumen,
4. mit anschließendem Abtrennen ungebundener Antikörper von den vorbestimmten Oberflächenabschnitten,
5. des Zugebens eines mit einer detektierbaren Markierung versehenen Reagenzes, das an Antikörper des Serums bindet, Entfernen von ungebundenem Reagenz von den vorbestimmten Oberflächenabschnitten,
6. des Detektierens der Markierung und des Bestimmens der vorbestimmten Oberflächenabschnitte,
7. mit anschließendem Zuordnen des Ergebnisses des Detektierens der Markierung zu den bestimmten Oberflächenabschnitten und
8. des Auswertens des Vorliegens oder der Abwesenheit der den bestimmten Oberflächenabschnitten zugeordneten detektierten Markierungen zur Bestimmung der Antikörper,
wobei die Oberflächenabschnitte der Träger jeweils einzelne Partikel sind, die einen individuellen, lichtmikroskopisch erkennbaren Code aufweisen, der aus einem Muster heller und dunkler Flächen besteht, und das Detektieren der Markierung und das Bestimmen der vorbestimmten Oberflächenabschnitte lichtmikroskopisch erfolgt.

2. Analyseverfahren nach Anspruch 1, **dadurch gekennzeichnet, dass** in Schritt 1. das Kontaktieren eines Aliquots der Serumprobe gleichzeitig mit zumindest drei unterschiedlichen Spezies von zumindest ein Blutgruppenantigen aufweisenden Zellen und/oder Membranfragmenten erfolgt, die zumindest ein Blutgruppenantigen aufweisen, deren jeweilige Blutgruppenantigene vorbestimmt sind und die jeweils auf vorbestimmten, individuellen Oberflächenabschnitten von Trägern immobilisiert sind.

3. Analyseverfahren nach Anspruch 2, **dadurch gekennzeichnet, dass** in Schritt 1 zumindest drei Spezies Erythrozytenmembranfragmente, deren jeweilige Antigene vorbestimmt sind und die jeweils auf vorbestimmten, individuellen Oberflächenabschnitten von Trägern immobilisiert sind, dadurch erzeugt werden, dass Erythrozyten auf vorbestimmten, individuellen Oberflächenabschnitten von Trägern immobilisiert werden, die vor, während oder nach dem Kontaktieren der Träger mit dem Aliquot der Serumprobe lysiert werden.

4. Analyseverfahren nach einem der voranstehenden Ansprüche, **dadurch gekennzeichnet, dass** die Markierung optisch detektierbar ist.

5. Analyseverfahren nach einem der voranstehenden Ansprüche, **dadurch gekennzeichnet, dass** die jeweils einzelnen Partikel an vorbestimmten, jeweils für die Partikel individuellen Stellen einer Unterlage angeordnet sind und das Detektieren der Markierung das Bestimmen der vorbestimmten Oberflächenabschnitte durch lichtmikroskopisches Detektieren der Markierung an den vorbestimmten Stellen der Unterlage erfolgt.

6. Analyseverfahren nach einem der voranstehenden Ansprüche, **dadurch gekennzeichnet, dass** das Auswerten durch Vergleichen des Vorliegens oder der Abwesenheit der detektierten Markierungen erfolgt, die den Codes für isolierte Blutgruppenantigene zugeordnet sind, mit dem Vorliegen oder der Abwesenheit der detektierten Markierungen, die den Codes für Erythrozytenmembranfragmente zugeordnet sind.

7. Analyseverfahren nach einem der voranstehenden Ansprüche, **dadurch gekennzeichnet, dass** ein isoliertes Blutgruppenantigen eine Spezies eines Blutgruppenantigens ist, die rekombinant hergestellt ist, aus menschlichen Zellen, insbesondere menschlichen Erythrozyten, gereinigt ist oder synthetisch hergestellt ist.

8. Analyseverfahren nach einem der voranstehenden Ansprüche, **dadurch gekennzeichnet, dass** zu einem weiteren Aliquot der Serumprobe zumindest ein Blutgruppenantigen in löslicher Form zugegeben wird, für das an dem vorbestimmten Oberflächenabschnitt das Vorliegen der Markierung gemessen wurde, und dass die Schritte 1 bis 8 wiederholt werden.

9. Analyseverfahren nach einem der voranstehenden Ansprüche, **dadurch gekennzeichnet, dass** vor Schritt 1 dem Serum zumindest eines der Blutgruppenantigene als lösliches Protein zugegeben wird, das aus der Gruppe ausgewählt ist, die Kn(a)/McC(a), Yt(a), Cr(a)/Dr(a), C4B^{∗}3, C4A^{∗}3, Sc1, JMH, Ch(a), Fy(a), Fy(b), Kell, Cellano, Do(a), Do(b), Lu(a), Lu(b), Xg(a), In(b), LW(a) umfasst oder daraus besteht.

10. Analyseverfahren nach einem der voranstehenden Ansprüche, **dadurch gekennzeichnet, dass** in Schritt 1 zumindest sechs unterschiedliche Erythrozytenmembranfragmente und in Schritt 2 zumindest sechs isolierte rekombinante Blutgruppenantigene jeweils auf vorbestimmten Oberflächenabschnitten von Trägern immobilisiert sind.

11. Analyseverfahren nach einem der voranstehenden Ansprüche, **dadurch gekennzeichnet, dass** das mit einer detektierbaren Markierung versehene Reagenz, das an Antikörper des Serums bindet, ein fluoreszenzmarkierter anti-human-Antikörper ist.

12. Analyseverfahren nach einem der voranstehenden Ansprüche, **dadurch gekennzeichnet, dass** beim Auswerten des Vorliegens oder der Abwesenheit der den Codes zugeordneten detektierten Markierungen zuerst das Vorliegen oder die Abwesenheit von Antikörpern gegen die isolierten Blutgruppenantigene bestimmt wird und anschließend das Vorliegen oder die Abwesenheit von Antikörpern gegen die Blutgruppenantigene bestimmt wird, die abzüglich der isolierten Blutgruppenantigene in Kombination auf den Erythrozytenmembranfragmenten vorliegen.

13. Analyseverfahren nach einem der voranstehenden Ansprüche, **dadurch gekennzeichnet, dass** als unspezifischer Hintergrund der Messwert von den detektierten Messwerten der Markierung eines Partikels abgezogen wird, der für eines von einem Paar antithetischer Antigene gemessen wird, wenn für das andere des Paar antithetischer Antigene ein ein signifikant höherer Messwert gemessen wird.

14. Analyseverfahren nach einem der Ansprüche 8 bis 13, **dadurch gekennzeichnet, dass** die Oberflächenabschnitte, auf denen Erythrozytenmembranfragmente oder Blutgruppenantigene immobilisiert sind, getrocknet sind und das als lösliches Protein zugegebene zumindest eine Blutgruppenantigen auf dem Träger aufgetrocknet ist.

## Claims

1. Analytical process for the determination of antibodies directed against blood group antigens in a serum sample comprising the steps of
1. contacting an aliquot of the serum sample concurrently with at least one species of cells having at least one blood group antigen and/or membrane fragments having at least blood group antigen, the respective blood group antigens being predetermined, and which are each immobilized on predetermined individual surface sections of carriers and
2. with at least one isolated blood group antigen which is immobilized on at least one predetermined individual surface section of carriers,
3. in a common liquid volume,
4. with subsequent separation of unbound antibodies from the predetermined surface sections,
5. adding a reagent provided with a detectable label, the reagent binding to an antibody of the serum, removing of unbound reagent from the predetermined surface sections,
6. detecting the label and determining the predetermined surface sections,
7. with subsequently correlating the result of detecting the label with the determined surface sections and
8. evaluating the presence or the absence of the detected labels correlated to the determined surface sections for determining the antibodies,
wherein the surface sections of carriers are each single particles that have an individual, light microscopically recognizable code consisting of a pattern of light and dark faces, and detecting the label and determining the predetermined surface sections occurs light-microscopically.

2. Analytical process according to claim 1, **characterized in that** in step 1 the contacting of the aliquot of the serum sample is concurrently with at least three different species of cells presenting at least one blood group antigen and/or membrane fragments presenting at least one blood group antigen, the respective blood group antigens of which are predetermined and which are respectively immobilized on predetermined individual surface sections of carriers.

3. Analytical process according to claim 2, **characterized in that** in step 1 at least three species of erythrocyte membrane fragments, the respective antigens of which are predetermined, and which are immobilized on predetermined individual surface sections of carriers are generated by immobilizing erythrocytes on predetermined individual surface sections of carriers which are lysed prior to, concurrent to or following the contacting of the carrier with the aliquot of the serum sample.

4. Analytical process according to one of the preceding claims, **characterized in that** the label is optically detectable.

5. Analytical process according to one of the preceding claims, **characterized in that** the respective single particles are arranged on sites of a base layer which are individual for the particles and detecting of the label is determining the predetermined surface sections by light microscopical detection of the label at the predetermined sites of the base layer.

6. Analytical process according to one of the preceding claims, **characterized in that** the evaluation is by comparing the presence or the absence of the detected labels which are correlated to the codes for isolated blood group antigens with the presence or the absence of the detected labels which are correlated to the codes for erythrocyte membrane fragments.

7. Analytical process according to one of the preceding claims, **characterized in that** an isolated blood group antigen is a species of a blood group antigen which is produced recombinantly, produced from human cells, especially from human erythrocytes, or is produced synthetically.

8. Analytical process according to one of the preceding claims, **characterized in that** to a further aliquot of the serum sample at least one blood group antigen is added in soluble form for which at the predetermined surface section the presence of the label was measured, and that the steps 1 to 8 are repeated.

9. Analytical process according to one of the preceding claims, **characterized in that** prior to step 1 at least one blood group antigen is added to the serum as a soluble protein, the blood group antigen being selected from the group comprising or consisting of Kn(a)/McC(a), Yt(a), Cr(a)/Dr(a), C4B^{∗}3, C4A^{∗}3, Sc1, JMH, Ch(a), Fy(a), Fy(b), Kell, Cellano, Do(a), Do(b), Lu(a), Lu(b), Xg(a), In(b), LW(a).

10. Analytical process according to one of the preceding claims, **characterized in that** in step 1 at least six different erythrocyte membrane fragments and in step 2 at least six isolated recombinant blood group antigens are immobilized each on predetermined surface sections of carrier.

11. Analytical process according to one of the preceding claims, **characterized in that** the reagent binding to an antibody of the serum and provided with a detectable label is a fluorescently labeled anti-human antibody.

12. Analytical process according to one of the preceding claims, **characterized in that** during the evaluation of the presence or absence of the detected labels correlated to the codes firstly the presence or the absence of antibodies against isolated blood group antigens is determined and subsequently the presence or the absence of antibodies against the blood group antigens is determined which, less the isolated blood group antigens, are present in combination on the erythrocyte membrane fragments.

13. Analytical process according to one of the preceding claims, **characterized in that** from the detected measurement values there is subtracted as a non-specific background the measurement value of a label of a particle which is measured for one of a pair of antithetic antigens if for the other of the pair of antithetic antigens a significantly higher measurement value is measured.

14. Analytical process according to one of claims 8 to 13, **characterized in that** the surface sections on which erythrocyte membrane fragments or blood group antigens are immobilized, are dried and that the at least one blood group antigen added as a soluble protein is dried onto the carrier.

## Revendications

1. Procédé d'analyse pour la détermination d'anticorps dirigés contre les antigènes des groupes sanguins dans un échantillon de sérum comprenant les étapes suivantes
1. la mise en contact simultanée d'une partie aliquote de l'échantillon de sérum avec au moins une espèce de cellules ayant au moins un antigène de groupe sanguin et/ou des fragments de membrane comprenant au moins un antigène de groupe sanguin, dont les antigènes de groupe sanguin respectifs sont prédéterminés et immobilisés chacun sur des parties de surface individuelles prédéterminées de supports, et
2. présentant au moins un antigène de groupe sanguin isolé immobilisé sur au moins une partie de surface individuelle prédéterminée de supports,
3. dans un volume liquide commun,
4. suivi de la séparation des anticorps non liés des parties de surface prédéterminées,
5. l'addition d'un réactif muni d'une étiquette détectable qui se lie aux anticorps du sérum, l'élimination du réactif non lié des parties de surface prédéterminées,
6. la détection de l'étiquette et la détermination des parties de surface prédéterminées,
7. l'association ultérieure du résultat de la détection de l'étiquette au niveau des parties de surface déterminées; et
8. l'évaluation de la présence ou de l'absence des étiquettes détectées associées aux parties de surface spécifiques pour la détermination des anticorps,
dans lequel les parties de surface des supports sont chacune des particules individuelles ayant un code individuel reconnaissable par microscopie optique consistant en un motif de surfaces claires et foncées, et dans lequel la détection de l'étiquette et la détermination des parties de surface prédéterminées sont réalisées par microscopie optique.

2. Procédé d'analyse selon la revendication 1, **caractérisé en ce que**, dans l'étape 1, la mise en contact d'une partie aliquote de l'échantillon de sérum est effectuée simultanément avec au moins trois espèces différentes de cellules et/ou fragments de membranes comprenant au moins un antigène de groupe sanguin, dont les antigènes du groupe sanguin sont prédéterminés et qui sont immobilisés sur des parties de surface individuelles prédéterminées de supports.

3. Procédé d'analyse selon la revendication 2, **caractérisé en ce que** dans l'étape 1, au moins trois espèces de fragments de membrane érythrocytaire dont les antigènes respectifs sont prédéterminés et qui sont immobilisés respectivement sur des parties de surface individuelle prédéterminée de supports sont produites en immobilisant des érythrocytes sur des parties de surface individuelle prédéterminée de supports qui sont lysés avant, pendant ou après la mise en contact des supports avec la partie aliquote de l'échantillon de sérum.

4. Procédé d'analyse selon l'une des revendications précédentes, **caractérisé en ce que** l'étiquette est optiquement détectable.

5. Procédé d'analyse selon l'une des revendications précédentes, **caractérisé en ce que** les particules individuelles respectives sont disposées à des emplacements prédéterminés d'un base, chaque emplacement étant individuel pour les particules, et la détection de l'étiquette consiste en la détermination des parties de surface prédéterminées par détection microscopique optique du marqueur aux emplacements prédéterminés de la base.

6. Procédé d'analyse selon l'une des revendications précédentes, **caractérisé en ce que** l'évaluation est réalisée en comparant la présence ou l'absence des étiquettes détectées associées aux codes des antigènes de groupes sanguins isolés avec la présence ou l'absence des étiquettes détectées associées aux codes des fragments de membrane érythrocytaire.

7. Procédé d'analyse selon l'une des revendications ci-dessus, **caractérisé en ce qu'**un antigène de groupe sanguin isolé est une espèce d'un antigène de groupe sanguin qui est produit par recombinaison, purifié à partir de cellules humaines, en particulier d'érythrocytes humains, ou produit par synthèse.

8. Procédé d'analyse selon l'une des revendications précédentes, **caractérisé en ce qu'**au moins un antigène de groupe sanguin sous forme soluble, pour lequel la présence de l'étiquette a été mesurée à la partie de surface prédéterminée, est ajouté à un autre aliquote de l'échantillon de sérum, et que les étapes 1 à 8 sont répétées.

9. Procédé d'analyse selon l'une des revendications précédentes, **caractérisé en ce qu'**avant l'étape 1, au moins un des antigènes du groupe sanguin est ajouté au sérum comme protéine soluble, choisi dans le groupe constitué de ou comprenant Kn(a)/McC(a), Yt(a), Cr(a)/Dr(a), C4B^{∗}3, C4A^{∗}3, Sei, JMH, Ch(a), Fy(a), Fy(b), Kell, Cellano, Do(a), Do(b), Do(a), Lu(b), Xg(a), In(b), LW(a).

10. Procédé d'analyse selon l'une des revendications ci-dessus, **caractérisé en ce que** dans l'étape 1, au moins six fragments de membrane érythrocytaire différents et dans l'étape 2, au moins six antigènes recombinants isolés de groupes sanguins sont immobilisés respectivement sur des parties de surface prédéterminées de supports.

11. Procédé d'analyse selon l'une des revendications ci-dessus, **caractérisé en ce que** le réactif muni d'une étiquette détectable, qui se lie aux anticorps du sérum, est un anticorps anti-humain marqué par fluorescence.

12. Procédé d'analyse selon l'une des revendications ci-dessus, **caractérisé en ce que**, lors de l'évaluation de la présence ou de l'absence des étiquettes détectées attribuées aux codes, la présence ou l'absence d'anticorps contre les antigènes isolé des groupes sanguins est d'abord déterminée et l'on détermine ensuite la présence ou l'absence des anticorps contre les antigènes de groupes sanguins qui sont présents en combinaison sur les fragments de membrane érythrocytaire moins les antigènes des groupes sanguins isolés.

13. Procédé d'analyse selon l'une des revendications précédentes, **caractérisé en ce qu'**en tant que fond non spécifique, la valeur mesurée est soustraite des valeurs mesurées détectées à partir de l'étiquette d'une particule mesurée pour l'un d'une paire d'antigènes antithétiques lorsqu'une valeur mesurée sensiblement supérieure est mesurée pour l'autre paire d'antigènes antithétiques.

14. Procédé d'analyse selon l'une quelconque des revendications 8 à 13, **caractérisé en ce que** les parties de surface, sur lesquelles des fragments de membrane érythrocytaire ou des antigènes de groupe sanguin sont immobilisés, sont séchées et ledit au moins un antigène de groupe sanguin ajouté comme protéine soluble est séché sur le support.
